(19) Europäisches Patentamt
European Patent Office
Office européen des brevets

(11) **EP 3 009 166 B1**

(12) **FASCICULE DE BREVET EUROPEEN**

(45) Date de publication et mention
de la délivrance du brevet:
**09.01.2019 Bulletin 2019/02**

(51) Int Cl.:
*A61N 1/36* *(2006.01)*   *A61N 1/362* *(2006.01)*

(21) Numéro de dépôt: **15185601.0**

(22) Date de dépôt: **17.09.2015**

(54) **DISPOSITIF MÉDICAL IMPLANTABLE ACTIF POUR LE TRAITEMENT DE L'INSUFFISANCE CARDIAQUE PAR STIMULATION DU NERF VAGUE**

AKTIVE IMPLANTIERBARE MEDIZINISCHE VORRICHTUNG ZUR BEHANDLUNG EINER HERZINSUFFIZIENZ DURCH STIMULATION DES VAGUSNERVS

ACTIVE IMPLANTABLE MEDICAL DEVICE FOR TREATING HEART FAILURE BY VAGUS NERVE STIMULATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.10.2014 FR 1459919**

(43) Date de publication de la demande:
**20.04.2016 Bulletin 2016/16**

(73) Titulaires:
• **Sorin CRM SAS**
 **92140 Clamart Cedex (FR)**
• **Université de Rennes 1**
 **35065 Rennes Cedex (FR)**

(72) Inventeurs:
• **BONNET, Jean-Luc**
 **91300 MASSY (FR)**
• **MULLEMEESTER, Mélanie**
 **92120 Montrouge (FR)**
• **HERNANDEZ, Alfredo**
 **35510 Cesson Sévigné (FR)**
• **CARRAULT, Guy**
 **35510 Cesson-Sévigné (FR)**

(74) Mandataire: **Grünecker Patent- und Rechtsanwälte PartG mbB Leopoldstraße 4 80802 München (DE)**

(56) Documents cités:
**EP-A1- 0 657 187   WO-A1-2014/074523**
**US-A1- 2012 095 530   US-A1- 2012 172 742**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les "dispositifs médicaux implantables actifs" tels que définis par la directive 90/385/CEE du 20 juin 1990 du Conseil des communautés européennes, plus précisément les implants permettant de délivrer des thérapies de stimulation du nerf vague ou de l'une de ses branches, dites thérapies VNS (*Vagus Nerve Stimulation*).

Elle concerne plus particulièrement l'utilisation de telles thérapies chez des patients en risque d'insuffisance cardiaque.

En effet, la stimulation du nerf vague agit sur les fonctions cardiovasculaires par réduction du rythme cardiaque et de la contractilité du myocarde avec diminution de la durée de la diastole, ce qui peut aider à réduire l'évolution d'un remodelage cardiaque susceptible de mener à une insuffisance cardiaque.

De façon générale, les patients en risque d'insuffisance cardiaque présentent un rythme cardiaque (HR, *Heart Rate*) accru par rapport aux patients sains, ce qui nécessite la prise de médicaments tels que des bêtabloquants pour de réduire le rythme cardiaque et améliorer ainsi le pronostic du patient.

Un rythme cardiaque élevé est souvent un prédicteur de mortalité accrue dans la population générale, avec un risque plus élevé d'accident cardiovasculaire, ce risque étant typiquement accru de 3 % pour chaque augmentation de 1 bpm du rythme cardiaque moyen.

On sait également que les thérapies VNS de stimulation du nerf vague ont un effet direct sur le rythme cardiaque, par action sur l'équilibre parasympathique. Cet effet de réduction du rythme cardiaque se manifeste :

- sur le court terme, par une modification de l'équilibre sympathovagal (SVB, *Sympatho-Vagal Balance*) entrainant un rétablissement d'un rythme cardiaque bien adapté à l'activité courante du patient,
- mais également sur le long terme avec des effets bénéfiques après plusieurs mois de thérapie VNS même si le rétablissement instantané de l'équilibre SVB n'est pas atteint.

L'une des difficultés de la thérapie VNS réside dans l'ajustement des paramètres de stimulation.

Sur le court terme, lorsqu'il s'agit de modifier de façon dynamique la stimulation en fonction de l'activité instantanée du patient, cette modification est opérée à partir de divers paramètres représentatifs de l'état courant du patient tels que rythme cardiaque, besoins métaboliques, etc. La thérapie VNS est alors automatiquement asservie ou modulée en fonction du niveau d'activité courant détecté, par exemple par sélection entre plusieurs niveaux d'énergie des impulsions de stimulation VNS.

En revanche, lorsque l'on souhaite ajuster les paramètres de la thérapie VNS pour obtenir un effet bénéfique sur le long terme, cet ajustement est en général laissé à l'appréciation du médecin, qui le fait de façon plus ou moins empirique.

D'autre part, l'ajustement ou le réajustement de ces paramètres par le médecin n'est effectué que lors des visites de contrôle, alors même que l'état du patient peut évoluer dans le temps, du fait de la thérapie VNS ou indépendamment de celle-ci, et que le paramétrage initial ne tient pas compte de ces évolutions.

Ainsi, des techniques telles que celles décrites par exemple par le US 2012/0095530 A1 (US 8 433 419 B2) permettent d'adapter la thérapie VNS au niveau d'activité du patient, ce niveau étant évalué par un capteur approprié tel qu'un accéléromètre intégré au boitier implanté du générateur d'impulsions VNS : le signal du capteur est comparé à une série de seuils prédéfinis et les paramètres de la stimulation VNS, principalement l'énergie de stimulation, sont sélectivement ajustés en fonction des seuils franchis, en particulier pour adapter la thérapie en laissant s'exprimer une accélération du rythme cardiaque corrélée à l'effort.

Toutefois, avec ce dispositif la modulation de la thérapie VNS ne tient pas compte de l'évolution à moyen et/ou long terme (par exemple d'un jour à l'autre et/ou sur une période de plusieurs jours ou semaines) de la pathologie dont est atteint le patient, aussi bien en cas d'aggravation que d'amélioration de son état général.

D'autres techniques comparables ont été proposées, mais il s'agit toujours d'opérer une adaptation dynamique de la thérapie VNS par asservissement sur un signal issu d'un capteur d'activité ou autre capteur d'un paramètre physiologique du patient, de façon dynamique pour améliorer l'efficacité instantanée de la thérapie VNS mais sans tenir compte de l'évolution sur le long terme de l'état du patient.

Le WO 2014/074523 A1 décrit un stimulateur VNS permettant la mémorisation de diverses données physiologiques et de stimulation, et intégrant des fonctions d'adaptation dans le temps de la stimulation VNS, contrôlées par exemple par une surveillance des arythmies induites par la thérapie, ou encore mettant en oeuvre une pondération des événements physiologiques mémorisés de manière à donner une plus grande importance aux événements récents par rapport aux plus anciens.

L'idée de base de l'invention est de proposer une technique permettant d'adapter automatiquement les paramètres de la thérapie VNS en fonction de l'activité physiologique du patient suivi sur une période de temps donnée, avec :

- une analyse rétrospective sur le long terme de cette activité, par exemple sur une durée d'un mois, et

- sur la base d'une comparaison entre :

  • le rythme cardiaque (HR) intrinsèque du patient, ci-après HRint, mesuré par le dispositif, et
  • un rythme cardiaque de référence, ci-après HRref, calculé en fonction de l'analyse rétrospective précitée à partir des données produites par au moins un capteur reflétant l'activité physique et/ou physiologique du patient - ce capteur ou combinaison de plusieurs capteurs étant simplement désigné ci-après "capteur d'activité".

Le rythme cardiaque intrinsèque HRint est déterminé à partir des intervalles RR mesurés, en excluant tous les cycles pathologiques (notamment ceux comprenant des extrasystoles), ceux comportant du bruit, ceux avec sur- ou sous-détection, etc.

Pour un patient normal, les valeurs du rythme de référence HRref calculées à partir du capteur d'activité sont très voisines de celles du rythme intrinsèque HRint.

En revanche, dans le cas d'une pathologie, tout particulièrement pour les patients en risque d'insuffisance cardiaque, le rythme intrinsèque HRint sera supérieur au rythme de référence HRref.

Toutefois, si l'état général du patient s'améliore, on fait l'hypothèse que :

- le rythme de référence HRref augmente du fait d'une meilleure activité physique, et simultanément
- le rythme intrinsèque HRint diminue du fait de l'amélioration de l'état du patient par rapport à sa pathologie, en partie du fait de la délivrance de la thérapie VNS.

Dès lors, les deux valeurs HRint et HRref vont avoir tendance à se rapprocher, conduisant à un diagnostic d'amélioration de l'état du patient. Inversement, si cet écart vient à augmenter, des alertes pourront être générées de manière que le médecin qui suit le patient puisse être prévenu sans attendre la prochaine visite.

Également, les paramètres de la stimulation VNS pourront être modifiés en fonction du diagnostic d'amélioration ou d'aggravation de l'état du patient.

- Plus précisément, l'invention propose un dispositif médical implantable actif pour le traitement de l'insuffisance cardiaque par stimulation VNS, comprenant de manière en elle-même connue, notamment d'après le WO 2014/074523 A1 précité : un générateur d'impulsions VNS et une électrode apte à être placée sur le nerf vague ou à proximité de celui-ci ; au moins un capteur d'activité, apte à délivrer des valeurs successives du niveau courant d'activité physique et/ou physiologique du patient ; des moyens de recueil de l'activité électrique du coeur, aptes à délivrer des valeurs successives du rythme cardiaque intrinsèque HRint courant du patient ; et des moyens de calcul d'un rythme cardiaque de référence, aptes à calculer des valeurs successives du rythme cardiaque de référence en fonction des valeurs respectives du niveau d'activité courant du patient.

De façon caractéristique de l'invention, le dispositif comprend en outre :

- des moyens de construction d'un premier histogramme, à partir desdites valeurs de rythme cardiaque de référence calculées par les moyens de calcul en fonction des valeurs respectives du niveau d'activité courant du patient délivrées par le au moins un capteur d'activité, sur une période de surveillance prédéfinie ;
- des moyens de construction d'un second histogramme, distinct du premier histogramme, à partir desdites valeurs de rythme cardiaque intrinsèque délivrées par les moyens de recueil de l'activité électrique du coeur sur ladite période de surveillance ; et
- des moyens comparateurs, aptes à comparer le premier et le second histogramme et à en dériver un indice représentatif de l'état du patient à l'issue de la période de surveillance.

Selon diverses caractéristiques subsidiaires avantageuses :

- les moyens comparateurs sont aptes à calculer, pour chacun des premier et second histogrammes, au moins un paramètre de l'histogramme, et à comparer le(s)dit(s) paramètre(s) respectif(s) pour dériver ledit indice représentatif de l'état du patient à l'issue de la période de surveillance ;
- ce paramètre de l'histogramme fait partie du groupe formé par : valeur maximale de l'histogramme, moyenne des valeurs de l'histogramme, médiane des valeurs de l'histogramme, aire sous la courbe enveloppe de l'histogramme, et les combinaisons de précédentes ;
- les moyens de calcul du rythme cardiaque de référence sont des moyens aptes à appliquer la relation suivante :

$$Fg = Basic_{Rate} + \left(G_{sample} - G_{low_{point}}\right)\frac{Max_{rate} - Basic_{rate}}{G_{high_{point}} - G_{low_{point}}}$$

Fg étant la valeur du rythme cardiaque de référence, $G_{sample}$ étant la valeur du niveau d'activité courant du patient délivré par le au moins un capteur d'activité, et $Basic_{rate}$, $Max_{rate}$, $G_{high_{point}}$ et $G_{low_{point}}$ étant des constantes prédéterminées ;

- les moyens de calcul du rythme cardiaque de référence sont des moyens aptes à opérer, en outre, un filtrage de la valeur du rythme cardiaque de référence calculée, par application d'une constante de temps aux valeurs de rythme cardiaque de référence successivement calculées ;
- le dispositif comprend en outre des moyens de contrôle, aptes à évaluer les variations dudit indice représentatif de l'état du patient sur une pluralité de périodes de surveillance successives ;
- les moyens de contrôle sont en outre aptes à modifier au moins un paramètre de fonctionnement du générateur VNS en fonction de la vérification ou non de critères prédéterminés par lesdites variations dudit indice représentatif de l'état du patient sur ladite pluralité de périodes de surveillance successives ;
- les moyens de contrôle sont en outre aptes à déclencher une alerte en fonction de la vérification ou non de critères prédéterminés par lesdites variations dudit indice représentatif de l'état du patient sur ladite pluralité de périodes de surveillance successives.

On va maintenant décrire un exemple de mise en oeuvre de la présente invention, en référence aux dessins annexés où les mêmes références désignent d'une figure à l'autre des éléments identiques ou fonctionnellement semblables.

La Figure 1 est une vue générale de présentation du dispositif de l'invention, montrant le générateur d'impulsions, le coeur et le nerf vague cervical ainsi que les sondes utilisées.

La Figure 2 est un schéma par blocs illustrant les principales fonctionnalités du générateur du dispositif de l'invention.

La Figure 3 est un histogramme, établi sur 24 heures, des valeurs moyennes d'activité du patient relevées au moyen d'un capteur accélérométrique.

La Figure 4 montre la fonction de transfert permettant d'obtenir une valeur cible de rythme cardiaque en fonction de la valeur de l'échantillon du signal G d'activité.

La Figure 5 montre la fonction de transfert permettant, avec application d'une constante de temps, d'obtenir le rythme cardiaque de référence à partir du rythme cardiaque cible.

La Figure 6 illustre la comparaison entre deux histogrammes obtenus respectivement à partir du rythme cardiaque intrinsèque mesuré et du rythme cardiaque de référence déterminé par le calcul.

La Figure 7 montre l'évolution sur le long terme des rythmes cardiaques intrinsèque et de référence dans le cas d'un patient dont l'état général ne varie pas de façon significative.

La Figure 8 est homologue de la Figure 7, pour un patient dont l'état général s'améliore du fait de l'application d'une thérapie VNS.

La Figure 9 est une représentation en coordonnées indépendantes du temps des deux paramètres de rythme cardiaque intrinsèque et de rythme cardiaque de référence dont l'évolution est représentée Figure 8.

Les Figures 10 et 11 sont homologues des Figures 8 et 9, dans le cas où la thérapie VNS initialement appliquée est modifiée en cours de période.

La Figure 12 est un organigramme montrant l'enchainement des différentes étapes de la première phase, de surveillance, de la technique de l'invention.

La Figure 13 est un organigramme montrant l'enchainement des différentes étapes de la seconde phase, d'ajustement de la thérapie VNS, de la technique de l'invention.

La Figure 14 est un organigramme montrant l'enchainement des différentes étapes de la troisième phase, de gestion des alertes, de la technique de l'invention.

On va maintenant décrire un exemple de mise en oeuvre de la technique selon l'invention.

En ce qui concerne ses aspects logiciels, l'invention peut être mise en oeuvre par une programmation appropriée du logiciel de commande d'un stimulateur VNS connu.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

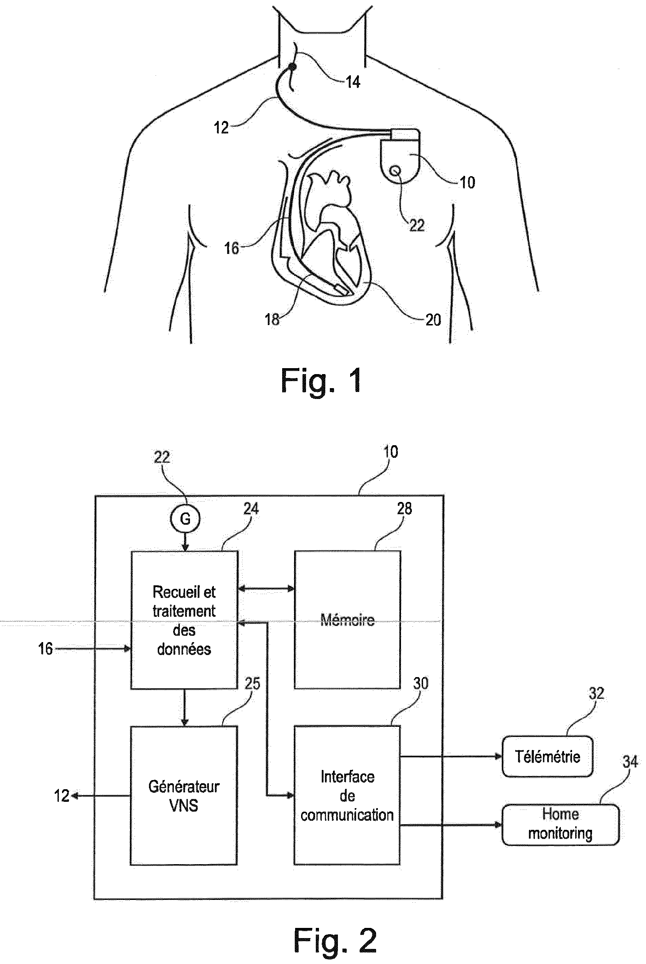

Sur la Figure 1, la référence 10 désigne le boitier d'un générateur d'impulsions. La stimulation du nerf vague est délivrée par une sonde 12 portant à sa partie distale une électrode implantée sur le nerf vague cervical 14 et susceptible de stimuler celui-ci par application de trains d'impulsions produits par le générateur 10. Cette technique de stimulation du système parasympathique n'est toutefois pas limitative, et d'autres moyens peuvent être employés à cet effet.

Le générateur 10 est également pourvu d'une sonde cardiaque 16 munie à son extrémité distale 18 d'une électrode de recueil de l'activité électrique du coeur 20. Cette sonde 16 recueille des signaux d'électrogramme endocavitaire (EGM) qui permettront de délivrer un signal représentatif de l'activité cardiaque du patient, en l'occurrence la fréquence cardiaque, déterminée par la durée des intervalles RR des dépolarisations cardiaques successives. Cette technique de recueil du rythme cardiaque n'est toutefois pas limitative, et d'autres moyens peuvent être employés à cet effet. Le boitier 10 est également muni d'un capteur 22 d'activité, notamment un capteur de mouvement du type accélérométrique ou "capteur G". Ici encore, ce type particulier de capteur n'est pas limitatif et d'autres modes de recueil d'un paramètre d'activité du patient peuvent être utilisés, par exemple un capteur physiologique de type ventilation-minute ou "capteur MV" donnant une indication du niveau d'activité du patient en fonction de ses besoins métaboliques mesurés à partir du rythme respiratoire et du volume respiratoire.

La Figure 2 illustre les principaux éléments du générateur 10.

Celui-ci comprend une unité 24 de recueil et de traitement des données produites par le capteur d'activité cardiaque (sonde 16) et du capteur d'activité (capteur G 22) permettant de déterminer et de calculer divers paramètres qui seront exposés ci-après. Ces paramètres pilotent un générateur VNS 26 délivrant des impulsions électriques à la sonde 12, impulsions dont l'énergie (stimulation en courant ou en tension par des impulsions de largeur contrôlée), la configuration en salves, la fréquence de récurrence, le rapport cyclique, etc. sont déterminés par l'unité 24.

Le générateur 10 comprend également une mémoire de données 28 permettant d'enregistrer les données calculées par l'unité 24 afin de pouvoir constituer des histogrammes et des séries comparatives de ces données sur le long terme.

Le générateur 10 est également pourvu d'une interface de communication 30 lui permettant d'échanger par télémétrie des données avec des dispositifs externes tels qu'un programmateur 32 et/ou un dispositif de *home monitoring* 34 permettant la télétransmission à un site distant des informations enregistrées dans la mémoire 28 pour assurer un suivi quotidien du patient depuis ce site distant.

On va maintenant décrire de façon détaillée la manière dont les signaux recueillis par les capteurs sont traités au sein du générateur 10 conformément aux enseignements de l'invention.

Ce traitement comprend trois phases successives, à savoir :

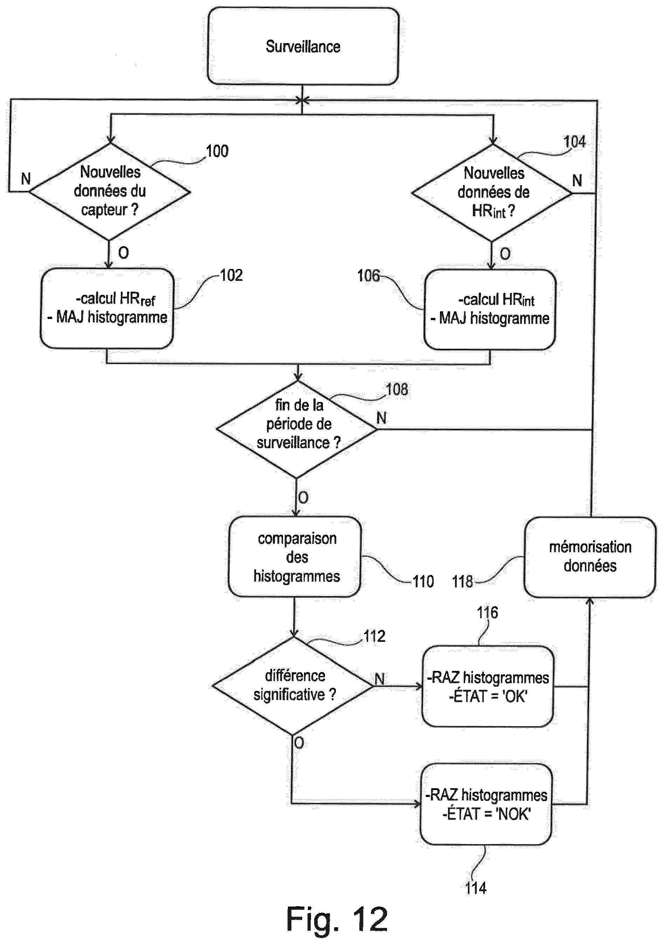

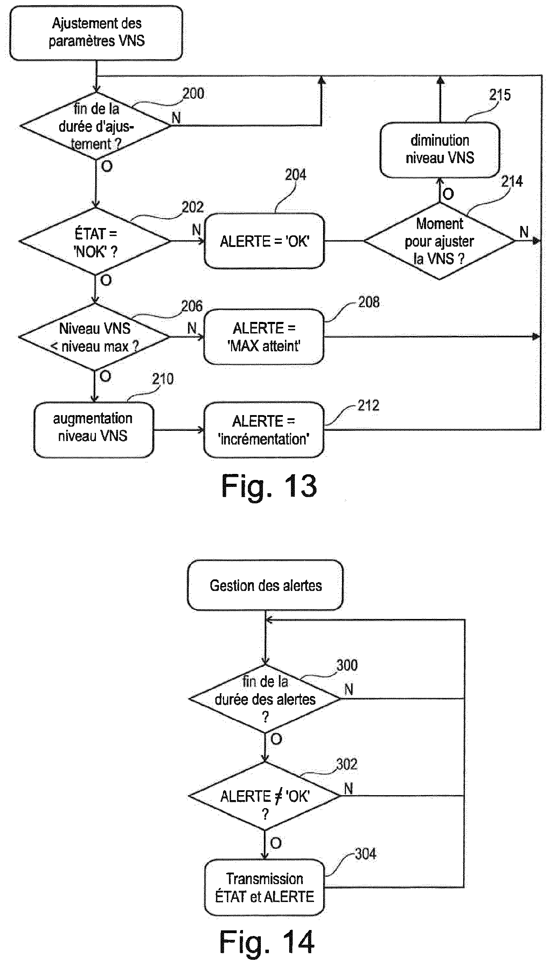

- une phase de surveillance du patient (dont le détail sera donné en référence à l'organigramme de la Figure 12), permettant de produire des séries de données de rythme cardiaque de référence HRref et de rythme cardiaque intrinsèque HRint, de mémoriser ces séries sur le long terme, d'établir des histogrammes et extraire de ces derniers des indicateurs de l'état du patient sur le long terme ;
- une phase d'ajustement des paramètres de la stimulation VNS (dont le détail sera donné en référence à l'organigramme de la Figure 13) en fonction des indicateurs d'état obtenus lors de la phase précédente ; et
- une phase de délivrance éventuelle d'alertes (dont le détail sera donné en référence à l'organigramme de la Figure 14), destinée à prévenir le médecin en cas d'aggravation de l'état du patient.

*Première phase : surveillance et évaluation de l'état du patient*

La période de surveillance ou *monitoring* est la période durant laquelle les données sont recueillies, traitées et analysées pour, ensuite, estimer l'évolution sur le long terme de l'état du patient. Cette période peut être fixe ou variable. Dans le présent exemple, cette période de surveillance est fixée à 24 heures mais elle pourrait prendre des valeurs différentes (une semaine, un mois...), ou varier en fonction du caractère plus ou moins critique de l'état du patient.

Pendant cette période de surveillance, deux paramètres sont mesurés de façon continue, à savoir le rythme cardiaque (HR) et le niveau d'activité du patient.

Si le niveau d'activité est mesuré par un capteur accélérométrique (capteur G), le signal est filtré pour ne conserver qu'une largeur de bande comprise typiquement entre 0,6 et 6 Hz afin d'éliminer la composante statique en fonction de la position du patient ainsi que les vibrations extérieures subies, en particulier lorsque le patient emprunte un mode de transport. Le signal filtré est ensuite moyenné, par exemple sur une période de Y = 6 s, avec pour résultat un échantillon Gmean de niveau moyen d'activité, délivré toutes les Y secondes.

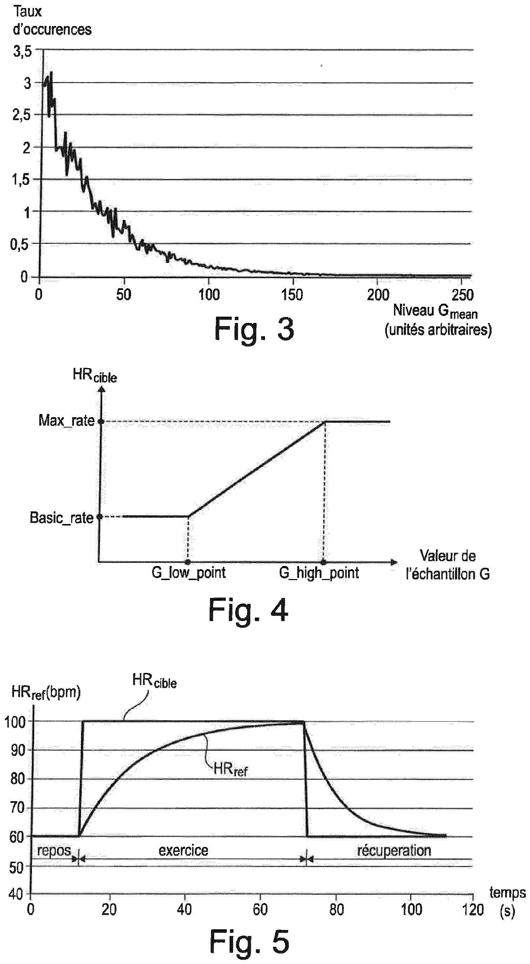

La distribution de ces valeurs d'activité Gmean sur une période par exemple de 24 heures est illustrée Figure 3.

De façon caractéristique de l'invention, cette valeur de niveau d'activité Gmean sert à déterminer une valeur de "rythme cardiaque de référence" HRref correspondant au rythme cardiaque attendu pour le patient si celui-ci était en bonne santé, pour son niveau d'activité.

Ce rythme cardiaque de référence HRref est calculé tous les X cycles cardiaques, typiquement X = 4 cycles, en fonction du dernier échantillon délivré par le capteur d'activité.

On applique à cet effet la relation linéaire suivante, qui définit la fonction de transfert du capteur G :

$$\mathrm{Fg} = \mathrm{Basic_{Rate}} + \left(\mathrm{G_{sample}} - \mathrm{G_{low_{point}}}\right)\frac{\mathrm{Max_{rate}} - \mathrm{Basic_{rate}}}{\mathrm{G_{high_{point}}} - \mathrm{G_{low_{point}}}}$$

Fg étant un rythme correspondant à une valeur cible $\mathrm{HR_{cible}}$,

$\mathrm{G_{sample}}$ étant la dernière valeur d'échantillon de signal délivrée par le capteur G, et

$\mathrm{Basic_{rate}}$, $\mathrm{G_{low_{point}}}$, $\mathrm{G_{high_{point}}}$ et $\mathrm{Max_{rate}}$ étant des constantes personnalisées, paramétrées par le médecin en fonction de l'activité physique propre à chaque patient ; en variante, ces valeurs peuvent être fixées à une valeur moyenne arbitraire, dès lors qu'elles ne sont pas modifiées au cours du temps.

La Figure 4 illustre la fonction de transfert $\mathrm{HR_{cible}} = f(\mathrm{G_{sample}})$ correspondant à la relation donnée plus haut.

$\mathrm{HR_{cible}}$, qui est calculée tous les quatre cycles cardiaques, est une donnée brute susceptible de variations rapides, par exemple au cours de cycles repos/activité/récupération, comme illustré sur la Figure 5.

Un filtrage supplémentaire est nécessaire pour reproduire le lissage naturel des variations du rythme cardiaque entre le repos et l'activité, par application d'une constante de temps selon une technique exposée par exemple dans le EP 0 657 187 A1 (ELA Medical). La valeur résultante est le rythme cardiaque de référence HRref qui sera utilisé dans la suite du traitement des données.

Sur la Figure 5, le patient est initialement dans une phase de repos, les deux valeurs HRcible et HRref étant à une valeur correspondant au rythme de base du patient. Lors de la phase d'exercice qui suit, le rythme cardiaque HRcible passe immédiatement à 100 bpm, tandis que le rythme cardiaque de référence HRref augmente lentement jusqu'à atteindre la valeur HRcible au bout d'une soixantaine de secondes. Lorsque l'exercice est terminé, HRcible retombe immédiatement à 60 bpm pendant la phase de récupération, tandis que le rythme de référence HRref diminue lentement jusqu'à cette valeur, au bout d'une trentaine de secondes.

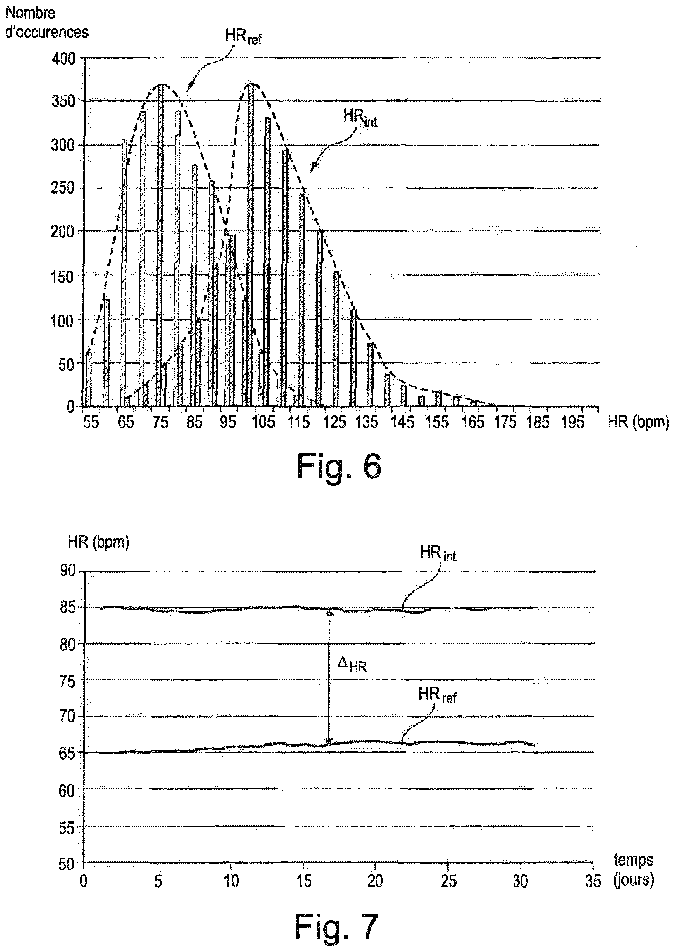

Un histogramme des valeurs de HRref ainsi calculées est établi sur la durée de la période de surveillance. La Figure 6 illustre cet histogramme. Parallèlement, au cours de la même période de surveillance, le rythme cardiaque intrinsèque du patient est mesuré, et un second histogramme est construit à partir des valeurs HRint ainsi obtenues.

Le rythme cardiaque intrinsèque est le rythme "normal-normal" déterminé à partir des intervalles RR détectés, en excluant les cycles anormaux (extrasystoles notamment) ou non significatifs (en particulier les cycles en bruit). On pourra se référer pour cette définition à :

[1] "Heart Rate Variability. Standards of Measurement, Physiological Interpretation, and Clinical use. Task Force of the European Society of Cardiology and the North American Society of Pacing and Electrophysiology", Eur Heart J, 17(3) : 354-381 (1996).

On notera que les deux histogrammes HRref et HRint sont constitués avec les mêmes paramètres statistiques de base, à savoir des *bins* de même durée, typiquement 5 bpm, et un échantillonnage effectué à une même périodicité, typiquement tous les quatre cycles cardiaques.

À la fin de chaque période de surveillance (typiquement, chaque jour), chacun des deux histogrammes HRref et HRint ainsi constitués est analysé, sur la base d'un ou plusieurs paramètres caractérisant l'histogramme, par exemple : valeur maximale atteinte, valeur moyenne, valeur médiane et/ou aire comprise sous la courbe, ou encore toute autre relation statistique reliant les deux histogrammes. Un indice quotidien représentatif est alors mémorisé, pour HRref et pour HRint.

La Figure 12 illustre sous forme d'organigramme les différentes étapes successives de cette phase de surveillance.

Lorsque le capteur G délivre de nouvelles données (bloc 100), le rythme de référence HRref est calculé de la manière indiquée plus haut (bloc 102) et l'histogramme correspondant est mis à jour.

Parallèlement, lorsque de nouvelles données de rythme cardiaque permettant le calcul du rythme intrinsèque sont disponibles (bloc 104), la valeur HRint est recalculée et l'histogramme correspondant est mis à jour (bloc 106).

À la fin de la période de surveillance (bloc 108) les deux histogrammes sont comparés (bloc 110), en fonction des différents paramètres qui peuvent les caractériser chacun (valeur moyenne, médiane, maximale, aire sous la courbe, test statistique, etc.) (bloc 110). Selon qu'une différence significative, c'est-à-dire dépassant un seuil donné, est constatée (bloc 112) un indicateur ÉTAT est positionné à 'OK' ou 'NOK' (blocs 114, 116), les histogrammes étant remis à zéro dans tous les cas (blocs 114, 116). Les données correspondant à HRref et HRint sont alors mémorisées (bloc 118) pour évaluation ultérieure.

*Deuxième phase : analyse de l'évolution de l'état du patient*

Les indices obtenus quotidiennement pour les valeurs HRref et HRint, qui reflètent l'état du patient à un jour donné, sont comparés de manière à évaluer leur variation relative sur le long terme.

La Figure 7 illustre un exemple d'évolution de ces indices sur une période d'environ un mois. On constate que, dans ce cas, l'écart $\Delta_{HR}$ entre les deux courbes HRref et HRint varie peu ou pas, indiquant que l'état du patient reste stationnaire.

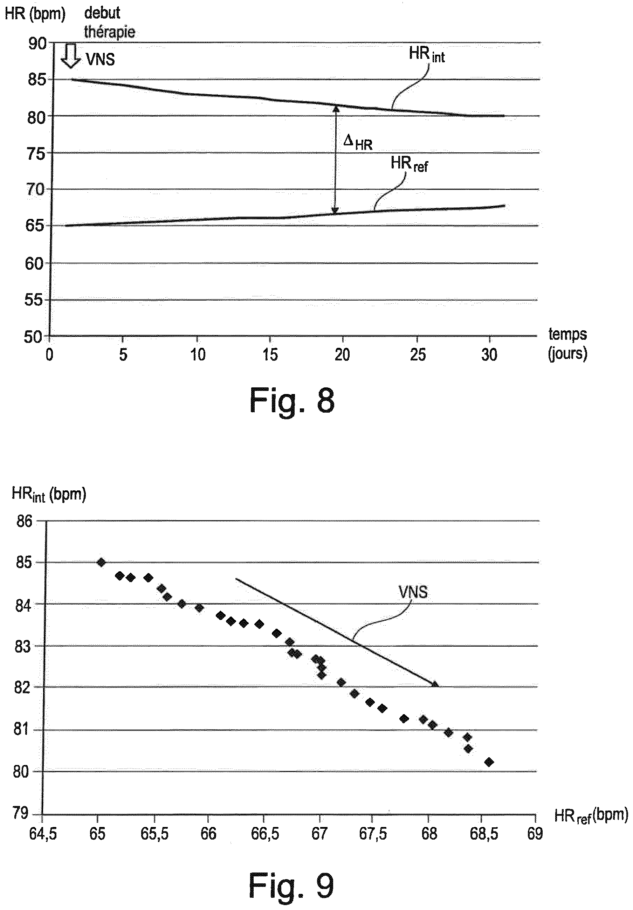

La Figure 8 est homologue de la Figure 7, après introduction d'une thérapie VNS poursuivie sur toute la durée de l'étude.

La thérapie VNS doit normalement augmenter HRref (sous l'effet de l'amélioration du patient pour réaliser des activités physiques) et diminuer HRint, ce que l'on constate effectivement sur les courbes de la Figure 8. On peut donc diagnostiquer une amélioration notable de l'état du patient sous l'effet de la thérapie VNS appliquée.

Dans une représentation fonctionnelle telle que celle illustrée Figure 9 (avec HRint en fonction de HRref), on constate que les deux paramètres sont associés de façon approximativement linéaire, avec une pente négative reflétant l'évolution favorable de l'état du patient sous l'effet de la thérapie appliquée (flèche "VNS").

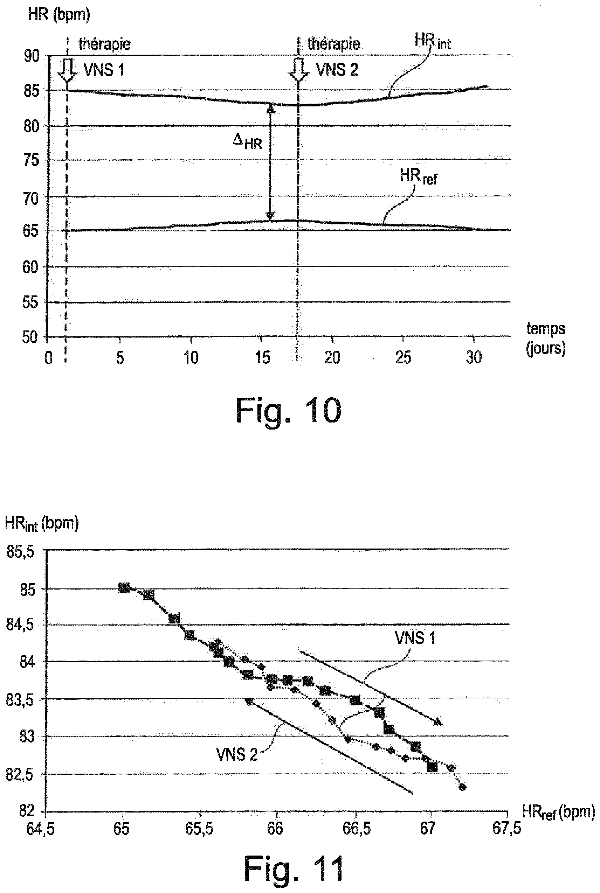

Les Figures 10 et 11 sont homologues des Figures 8 et 9, dans une situation où la thérapie VNS initialement appliquée (VNS1, par exemple une stimulation par des salves de 4 impulsions de 3 mA, de 0,24 ms de largeur, avec un rapport cyclique de 1/1 et un séquencement de 10"/30") a été modifiée après une quinzaine de jours (VNS2, par exemple une stimulation par des salves de 2 impulsions de 2 mA, de 0,24 ms de largeur, avec un rapport cyclique de 1/1 et un séquencement de 10"/30"), par exemple avec augmentation/diminution de l'énergie de stimulation, modification de la fréquence et/ou du contenu des salves d'impulsions, de leur rapport cyclique, etc.

On constate alors que, après avoir convergé (amélioration de l'état du patient), les deux courbes viennent à diverger, ceci reflétant une dégradation de l'état du patient après que la thérapie a été modifiée de VNS1 et VNS2.

Sur la représentation fonctionnelle de la Figure 11, les carrés illustrent les valeurs successives relevées au cours de la première période, tandis que les losanges illustrent celles relevées au cours de la seconde période. On constate une inversion de sens (entre la flèche "VNS1" et la flèche "VNS2"), reflétant dans la seconde période une dégradation de l'état du patient du fait de la nouvelle thérapie.

En réponse, les paramètres de la thérapie VNS peuvent être réajustés de manière à retourner à une configuration permettant l'amélioration de l'état du patient.

Ce reparamétrage peut être effectué de façon périodique, par exemple hebdomadaire, jusqu'à ce que l'écart entre les deux courbes HRref et HRint diminue au-dessous d'un seuil prédéfini (HRref, qui une valeur calculée sur la base de l'activité du patient, représentant l'idéal vers lequel HRint doit converger).

La périodicité de l'ajustement des paramètres VNS peut être la même que la période de surveillance (par exemple quotidienne), ou bien plus longue que la période de surveillance (par exemple hebdomadaire) de manière à ajuster plus lentement la thérapie VNS.

La Figure 13 illustre sous forme d'organigramme les différentes étapes de cette phase d'ajustement des paramètres VNS.

À la fin de la durée prédéterminée de cette phase d'ajustement (bloc 200), l'indicateur ÉTAT est testé (bloc 202). Si celui-ci est à la valeur 'OK', un paramètre ALERTE est alors positionné à 'OK', signifiant que l'état du patient est conforme et n'appelle pas de déclenchement d'alerte. Dans ce cas, la thérapie VNS doit être réévaluée à intervalles réguliers (bloc 214). Si ce moment est atteint, par exemple après 7 jours consécutifs à 'OK', le niveau de la stimulation VNS est réduit (bloc 216) : par exemple, le nombre d'impulsions VNS de chaque salve est réduit d'une unité, et si une limite inférieure est atteinte, par exemple deux impulsions par salve, alors le courant délivré est diminué d'un pas (typiquement de 0,5 mA).

Si l'indicateur ÉTAT est à 'NOK', alors le dispositif examine si le niveau de la stimulation VNS est à son maximum (bloc 206). Dans l'affirmative, le paramètre ALERTE est positionné à 'MAXIMUM ATTEINT', pour signifier que, bien que l'état du patient soit satisfaisant, la stimulation VNS a atteint le niveau maximal qu'il était possible de délivrer (bloc 208). Dans le cas contraire, le niveau de la stimulation VNS est augmenté (bloc 210) et le paramètre ALERTE est positionné à 'INCRÉMENTATION' (bloc 212) pour signifier qu'il a été nécessaire d'appliquer au patient une thérapie VNS plus forte : par exemple, le nombre d'impulsions VNS de chaque salve est augmenté d'une unité, et si une limite supérieure est atteinte, par exemple de quatre impulsions par salve, alors le courant délivré est augmenté d'un pas (typiquement de 0,5 mA).

Cependant, pour diminuer ou augmenter l'effet sur le rythme cardiaque, tout autre paramètre peut être modulé pour atteindre l'effet souhaité (fréquence des impulsions, délai QRS-salve VNS, ...).

*Troisième phase : gestion des alertes*

Si les courbes HRref et HRint viennent à s'écarter (comme dans la seconde période illustrée Figure 10), il peut être souhaitable d'avertir le médecin sans attendre la visite de suivi, par exemple en générant une alarme et en transmettant celle-ci à un site distant via un système de *home monitoring.*

La Figure 14 illustre, sous forme d'organigramme, les différentes étapes de la troisième phase de gestion des alertes.

À la fin de la durée prédéterminée de gestion des alertes (bloc 300), par exemple quotidiennement, l'indicateur ALERTE est examiné (bloc 302). S'il n'est pas à la valeur 'OK', alors les valeurs des indicateurs ÉTAT et ALERTE sont télétransmis (bloc 304) pour analyse et action éventuelle par le praticien, typiquement au moyen d'un système de *home monitoring* relié à un site distant.

**Revendications**

**1.** Un dispositif médical implantable actif (10) pour le traitement de l'insuffisance cardiaque par stimulation du nerf vague ou de l'une de ses branches, VNS, comprenant :

- un générateur d'impulsions VNS (26) et une électrode (12) apte à être placée sur ou à proximité du nerf vague d'un patient ;
- au moins un capteur d'activité (22), apte à délivrer des valeurs successives du niveau courant d'activité physique et/ou physiologique du patient ;
- un capteur d'activité cardiaque (16), apte à recueillir l'activité électrique du coeur et à délivrer un signal représentatif de l'activité cardiaque (HR) du patient ; et
- une unité (24) de recueil et de traitement des données produites par le capteur d'activité cardiaque (16) et ledit au moins un capteur d'activité (22), ladite unité (24) étant configurée pour :

- recueillir le signal représentatif de l'activité cardiaque (HR) issu du capteur d'activité cardiaque (16) et déterminer des valeurs successives d'un rythme cardiaque intrinsèque (HRint) courant du patient à partir du signal représentatif de l'activité cardiaque (HR) recueilli ; et
- calculer des valeurs successives d'un rythme cardiaque de référence (HRref), ledit rythme cardiaque de référence (HRref) correspondant au rythme cardiaque attendu pour le patient s'il était en bonne santé, en fonction des valeurs successives respectives du niveau d'activité courant du patient délivrées par ledit au moins un capteur d'activité (22),

**caractérisé en ce que**

- l'unité (24) de recueil et de traitement des données produites par le capteur d'activité cardiaque (16) et ledit au moins un capteur d'activité (22) est en outre configurée pour :

- construire un premier histogramme, à partir desdites valeurs successives de rythme cardiaque de référence (HRref) en fonction des valeurs respectives du niveau d'activité courant du patient délivrées par ledit au moins un capteur d'activité (22), sur une période de surveillance prédéfinie ;
- construire un second histogramme, distinct du premier histogramme, à partir desdites valeurs de rythme cardiaque intrinsèque (HRint), sur ladite période de surveillance prédéfinie ; et
- comparer le premier et le second histogramme et à en dériver un indice ($\Delta_{HR}$) représentatif de l'état du patient à l'issue de ladite période de surveillance prédéfinie ;

et **en ce que**

- ladite unité (24) est en outre configurée pour appliquer la relation suivante :

$$Fg = Basic_{Rate} + \left(G_{sample} - G_{lowpoint}\right)\frac{Max_{rate} - Basic_{rate}}{G_{highpoint} - G_{lowpoint}}$$

Fg étant la valeur du rythme cardiaque de référence,

$G_{sample}$ étant la valeur du niveau d'activité courant du patient délivré par ledit au moins un capteur d'activité (22), et

$Basic_{rate}$, $Max_{rate}$, $G_{highpoint}$ et $G_{lowpoint}$ étant des constantes prédéterminées.

2. Le dispositif de la revendication 1, dans lequel ladite unité (24) est en outre configurée pour :

calculer, pour chacun des premier et second histogrammes, au moins un paramètre de l'histogramme, et comparer le(s)dit(s) paramètre(s) respectif(s) pour dériver ledit indice ($\Delta_{HR}$) représentatif de l'état du patient à l'issue de la période de surveillance.

3. Le dispositif de la revendication 2, dans lequel ledit au moins un paramètre de l'histogramme est un paramètre du groupe formé par : valeur maximale de l'histogramme, moyenne des valeurs de l'histogramme, médiane des valeurs de l'histogramme, aire sous la courbe enveloppe de l'histogramme, et les combinaisons de précédentes.

4. Le dispositif de la revendication 1, dans lequel ladite unité (24) est en outre configurée pour opérer, en outre, un filtrage de la valeur du rythme cardiaque de référence (HRref) calculée, par application d'une constante de temps aux valeurs de rythme cardiaque de référence (HRref) successivement calculées.

5. Le dispositif de la revendication 1, dans lequel ladite unité (24) est en outre configurée pour évaluer les variations dudit indice représentatif de l'état du patient sur une pluralité de périodes de surveillance successives.

6. Le dispositif de la revendication 5, dans lequel ladite unité (24) est en outre configurée pour modifier au moins un paramètre de fonctionnement du générateur VNS (200-212) en fonction de la vérification ou non de critères prédéterminés par lesdites variations dudit indice représentatif de l'état du patient sur ladite pluralité de périodes de surveillance successives.

7. Le dispositif de la revendication 5, dans lequel ladite unité (24) est en outre configurée pour déclencher une alerte (300-304) en fonction de la vérification ou non de critères prédéterminés par lesdites variations dudit indice ($\Delta_{HR}$) représentatif de l'état du patient sur ladite pluralité de périodes de surveillance successives.

**Patentansprüche**

1. Aktive implantierbare medizinische Vorrichtung (10) zur Behandlung einer Herzinsuffizienz durch Stimulation des Vagusnervs oder eines seiner Äste, VNS, umfassend:

- einen VNS-Impulsgenerator (26) und eine Elektrode (12), die dazu geeignet ist, auf oder in der Nähe des Vagusnervs eines Patienten angebracht zu werden;
- mindestens einen Aktivitätssensor (22), der dazu geeignet ist, aufeinanderfolgende Werte der aktuellen körperlichen und/oder physiologischen Aktivität des Patienten auszugeben;
- einen Herzaktivitätssensor (16), der dazu geeignet ist, die elektrische Aktivität des Herzens zu erfassen und ein die Herzaktivität (HR) des Patienten darstellendes Signal auszugeben; und
- eine Einheit (24) zur Erfassung und Bearbeitung von durch den Herzaktivitätssensor (16) und den mindestens einen Aktivitätssensor (22) erzeugten Daten, wobei die Einheit (24) dazu ausgebildet ist:

- das vom Herzaktivitätssensor (16) ausgegebene, die Herzaktivität (HR) darstellende Signal zu erfassen und aus dem die Herzaktivität (HR) darstellenden Signal aufeinanderfolgende Werte eines aktuellen intrinsischen Herzrhythmus (HRint) eines Patienten zu bestimmen; und
- in Abhängigkeit der durch den mindestens einen Aktivitätssensor (22) ausgegebenen jeweiligen aufeinanderfolgenden Werte der aktuellen Aktivität des Patienten, aufeinanderfolgende Werte eines Referenz-Herzrhythmus (HRref) auszurechnen, wobei der Referenz-Herzrhythmus (HRref) einem in einem gesunden Patienten zu erwartenden Herzrhythmus entspricht,

**dadurch gekennzeichnet, dass**

- die Einheit (24) zur Erfassung und Bearbeitung der durch den Herzaktivitätssensor (16) und den mindestens einen Aktivitätssensor (22) erzeugten Daten außerdem dazu konfiguriert ist:

- aus den aufeinanderfolgenden Werten des Referenz-Herzrhythmus (HRref), in Abhängigkeit der durch den mindestens einen Aktivitätssensor (22) ausgegebenen jeweiligen Werte der aktuellen Aktivität des Patienten, in einem vorbestimmten Überwachungszeitraum, ein erstes Histogramm zu erstellen;
- aus den Werten des intrinsischen Herzrhythmus (HRint) in dem vorbestimmten Überwachungszeitraum ein vom ersten Histogramm unterschiedliches zweites Histogramm zu erstellen; und
- das erste und das zweite Histogramm miteinander zu vergleichen und daraus einen den Zustand des Patienten darstellenden Index ($\Delta_{HR}$) nach Ablauf des vorbestimmten Überwachungszeitraums abzuleiten;

und dass

- die Einheit (24) außerdem dazu konfiguriert ist, Folgende Gleichung anzuwenden:

$$Fg = Basic_{Rate} + \left( G_{Sample} - G_{low_{point}} \right) \frac{Max_{rate} - Basic_{rate}}{G_{high_{point}} - G_{low_{point}}}$$

wobei Fg der Wert des Referenz-Herzrhythmus ist,
$G_{sample}$ der durch den mindestens einen Aktivitätssensor (22) ausgegebene Wert der aktuellen Aktivität des Patienten ist, und
$Basic_{rate}$, $Max_{rate}$, $G_{highpoint}$ und $G_{lowpoint}$ vorbestimmte Festwerte sind.

2. Vorrichtung nach Anspruch 1, wobei die Einheit (24) außerdem dazu konfiguriert ist:

für jedes der ersten und zweiten Histogramme mindestens einen Parameter des Histogramms auszurechnen, und
den/die jeweiligen Parameter zu Vergleichen, um daraus den den Zustand des Patienten darstellenden Index ($\Delta_{HR}$) nach Ablauf des vorbestimmten Überwachungszeitraums abzuleiten.

3. Vorrichtung nach Anspruch 2, wobei der mindestens eine Parameter des Histogramms ein unter folgenden Parametern ausgewählter Parameter ist: Maximalwert des Histogramms, Durchschnitt der Werte des Histogramms, Medianwert der Werte des Histogramms, Fläche unter der Hüllkurve des Histogramms, und deren Kombinationen.

4. Vorrichtung nach Anspruch 1, wobei die Einheit (24) außerdem dazu konfiguriert ist, außerdem eine Filterung des ausgerechneten Wertes des Referenz-Herzrhythmus (HRref) durchzuführen, indem eine Zeitkonstante auf die aufeinanderfolgend ausgerechneten Werte des Referenz-Herzrhythmus (HRref) angewendet wird.

5. Vorrichtung nach Anspruch 1, wobei die Einheit (24) außerdem dazu konfiguriert ist, die Variationen des den Zustand des Patienten darstellenden Indexes über eine Vielzahl von aufeinanderfolgenden Überwachungszeiträumen zu beurteilen.

6. Vorrichtung nach Anspruch 5, wobei die Einheit (24) außerdem dazu konfiguriert ist, mindestens einen Betriebsparameter des VNS-Generators (200-212) zu ändern, je nachdem, ob vorbestimmte Kriterien durch die Variationen des den Zustand des Patienten darstellenden Indexes über die Vielzahl von aufeinanderfolgenden Überwachungszeiträumen verifiziert werden oder nicht.

7. Vorrichtung nach Anspruch 5, wobei die Einheit (24) außerdem dazu konfiguriert ist, einen Alarm (300-304) auszulösen, je nachdem, ob vorbestimmte Kriterien durch die Variationen des den Zustand des Patienten darstellenden Indexes ($\Delta_{HR}$) über die Vielzahl von aufeinanderfolgenden Überwachungszeiträumen verifiziert werden oder nicht.

Claims

1.  An active implantable medical device (10) for treating heart failure by stimulating the vagus nerve or one of its branches, VNS, comprising:

    - a VNS pulse generator (26) and an electrode (12) adapted to be placed on or in the vicinity of the vagus nerve of a patient;
    - at least one activity sensor (22) adapted to issue successive values of the current level of physical and/or physiological activity of the patient;
    - a cardiac activity sensor (16) adapted to capture the electrical activity of the heart and to issue a signal representing the cardiac activity (HR) of the patient; and
    - a unit (24) for capturing and treating data produced by the cardiac activity sensor (16) and said at least one activity sensor (22), said unit (24) being configured to:

        - capture the signal representing the cardiac activity (HR) issued by the cardiac activity sensor (16) and determine successive values of a current intrinsic heart rate (HRint) of the patient based on the captured signal representing the cardiac activity (HR); and
        - calculate successive values or a reference heart rate (HRref), said reference heart rate (HRref) corresponding to the heart rate to be expected in a healthy patient, based on the respective successive values of the current activity level of the patient issued by said at least one activity sensor (22),

    **characterized in that**

        - the unit (24) for capturing and treating the data produced by the cardiac activity sensor (16) and said at least one activity sensor (22) is further configured to:

            - construct a first histogram based on said successive values of the reference heart rate (HRRef) as a function of the respective values of the current activity level of the patient issued by said at least one activity sensor (22) over a predefined monitoring time period;
            - construct a second histogram, different from the first histogram, based on said values of the intrinsic heart rate (HRint) over the predefined monitoring time period; and
            - compare the first and the second histogram et derive an index ($\Delta_{HR}$) representing the patient's condition at the end of said predefined monitoring time period;

    and **in that**

        - said unit (24) is further configured to apply the following equation:

$$Fg = Basic_{Rate} + \left(G_{Sample} - G_{low_{point}}\right) \frac{Max_{rate} - Basic_{rate}}{G_{high_{point}} - G_{low_{point}}}$$

    wherein Fg is the value of the reference heart rate,
    $G_{sample}$ is the value of the current activity level of the patient issued by said at least one activity sensor (22), and
    $Basic_{rate}$, $Max_{rate}$, $G_{highpoint}$ and $G_{lowpoint}$ are predetermined constants.

2.  The device according to claim 1, wherein said unit (24) is further configured to:

    calculate, for each of the first and second histograms, at least one parameter of the histogram, and
    compare said respective parameter(s) in order to derive said index ($\Delta_{HR}$) representing the patient's condition at the end of the monitoring time period.

3.  The device according to claim 2, wherein said at least one parameter of the histogram is a parameter chosen among the group comprising: a maximum value of the histogram, an average of the values of the histogram, a median value of the values of the histogram, an area under the envelope curve of the histogram, and combinations thereof.

4. The device according to claim 1, wherein said unit (24) is further configured to carry out a filtering of the calculated value of the reference heart rate (HRref) by applying a time constant to the successively calculated values of the reference heart rate (HRref).

5. The device according to claim 1, wherein said unit (24) is further configured to evaluate the variations of said index representing the patient's condition over a plurality of successive monitoring time periods.

6. The device according to claim 5, wherein said unit (24) is further configured to modify at least one operating parameter of the VNS generator (200-212) according to whether predetermined criteria are verified or not by said variations of said index representing the patient's condition over said plurality of successive monitoring time periods.

7. The device according to claim 5, wherein said unit (24) is further configured to trigger an alert (300-304) according to whether predetermined criteria are verified or not by said variations of said index ($\Delta_{HR}$) representing the patient's condition over said plurality of successive monitoring time periods.

Fig. 1

Fig. 2

Taux
d'occurences

Fig. 3

Fig. 4

Fig. 5

Fig. 6

Fig. 7

Fig. 8

Fig. 9

Fig. 10

Fig. 11

# Fig. 12

Fig. 13

Fig. 14

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120095530 A1
- US 8433419 B2
- WO 2014074523 A1
- EP 0657187 A1

**Littérature non-brevet citée dans la description**

- dispositifs médicaux implantables actifs. Conseil des communautés européennes, 20 Juin 1990
- Heart Rate Variability. Standards of Measurement, Physiological Interpretation, and Clinical use. Eur Heart J. Task Force of the European Society of Cardiology, 1996, vol. 17, 354-381